# EUROPEAN PATENT APPLICATION

(11) **EP 4 029 526 A1**
(43) Date of publication of application: **20.07.2022**
(21) Application number: 21151283.5
(22) Date of filing: 13.01.2021
(51) Int. Cl.: A61L 2/10, A61L 2/00, A61L 2/24, E03D 9/00, A47K 13/00

(54) **SETTING A STATE OF UV RADIATION SOURCES IN DEPENDENCE ON INPUT FROM A DETECTOR ARRANGEMENT**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); Niessen, Eduard Matheus Johannes, 5656 AE Eindhoven (NL); Martens, Peter, 5656 AE Eindhoven (NL)
(74) Representative: Philips Intellectual Property & Standards

(57) **Abstract**

A system 1 comprises a radiation body 10, a radiation arrangement 20 configured to provide radiation at least comprising UV radiation 23 to the radiation body 10, a detector arrangement 30 configured to detect an internal radiation intensity of radiation (25) in the radiation body 10 at at least one detector position, and a controller arrangement 40 configured to control the radiation arrangement 20 in dependence on input from the detector arrangement 30. When the input from the detector arrangement 30 indicates a change of the internal radiation intensity that can be assumed to follow from an event of touch on a radiation exit window 11 of the radiation body 10, an action of temporarily changing a state of at least one UV radiation source 21 of the radiation arrangement 20 from a maintenance state to a disinfecting state of increased intensity of the UV radiation 23 is performed.

## Description

### FIELD OF THE INVENTION

The invention relates to a system comprising a radiation body comprising a radiation exit window, wherein the radiation body is configured to receive radiation that at least comprises UV radiation, and to radiate at least part of the radiation to the exterior of the radiation body via the radiation exit window; a radiation arrangement configured to provide the radiation, comprising at least one UV radiation source configured to emit UV radiation; a detector arrangement configured to detect an internal radiation intensity of radiation in the radiation body at at least one detector position; and a controller arrangement, functionally coupled to the radiation arrangement and the detector arrangement.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element, an optical sensor and a control system. The waveguide element comprises a radiation exit window, wherein the waveguide element is i) configured to receive radiation, wherein the radiation at least comprises UV radiation, ii) configured to radiate at least part of the radiation to the exterior of the waveguide element via the radiation exit window, and iii) configured to internally reflect part of the radiation at the radiation exit window. The optical sensor is configured to sense an internal reflection intensity of the internally reflected radiation. The control system is functionally coupled to the optical sensor and is configured to reduce the intensity of the radiation as function of reaching a predetermined first threshold of a reduction of the internal reflection intensity over time.

WO 2018/215272 A1 teaches that the UV radiation is used for killing microorganisms as may be present on the radiation exit window, or for rendering the microorganisms inactive or unable to reproduce. As a practical example of microorganisms, bacteria are mentioned. The disinfecting effect of using UV radiation is mainly governed by a total dose of the UV radiation. In the context of using UV radiation, it may be necessary to take specific measures when higher organisms, including human beings, may be in a position of receiving the UV radiation, which is especially the case if it is possible for the higher organisms to physically contact radiation emitting surfaces.

During operation of the known system, radiation is coupled out of the waveguide element through the radiation exit window if some object touches the window. This outcoupling means that less radiation will stay inside the waveguide element and is also referred to as frustration of the (total) internal reflection. Therefore, by using the optical sensor to monitor an internal reflection intensity of the internally reflected radiation, it is possible to detect a situation of some object touching the window, especially some object that is considerably larger than microorganisms, such as a human hand or finger. When a considerable step is observed in the signal provided by the optical sensor, it is assumed that this means that a relatively large object contacts the window. In order to ensure safety in such a situation, it may be determined that the radiation needs to be shut off, at least temporarily.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications of the invention that is the subject of WO 2018/215272 A1, it is important that external surfaces are kept in a disinfected state while situations in which the UV radiation used in the process might cause harm to higher organisms such as human beings need to be avoided.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. In the context of the present invention, it is acknowledged that operating the system requires a supply of electric power and that this renders the system not very well suitable for use in contexts in which only a limited amount of power is available, such as contexts in which there cannot be a connection to the mains.

### SUMMARY OF THE INVENTION

It is an object of the invention to design a system comprising a radiation body and a radiation arrangement in such a way that a possibility of effectively obtaining disinfection results at the position of a radiation exit window of the radiation body at only a minimum amount of power is provided. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation, and to radiate at least part of the radiation to the exterior of the radiation body via the radiation exit window; a radiation arrangement configured to provide the radiation, comprising at least one UV radiation source configured to emit UV radiation; a detector arrangement configured to detect an internal radiation intensity of radiation in the radiation body at at least one detector position; and a controller arrangement, functionally coupled to the radiation arrangement and the detector arrangement, configured to set a normal state of the at least one UV radiation source as a default, in which state the at least one UV radiation source provides the UV radiation with an intensity at a maintenance level, and configured to receive input from the detector arrangement and to apply a controller algorithm that involves i) determining at least one safety test value that is representative of a parameter of the internal radiation intensity detected by the detector arrangement at the at least one detector position, ii) assessing whether the at least one safety test value is inside or outside of a safety range of values associated with the at least one detector position, and iii) after occurrence of a disturbance incident involving a shift of the at least one safety test value from inside the safety reference range of values to outside of the safety reference range of values and a subsequent restoration incident involving a shift of the at least one test value in opposite direction, temporarily setting a disinfecting state of the at least one UV radiation source, in which state the at least one UV radiation source provides the UV radiation with an intensity at a level that is increased relative to the maintenance level.

It follows from the foregoing that the system according to the invention comprises i) a radiation body comprising a radiation exit window, wherein the radiation body is configured to receive radiation that at least comprises UV radiation, and to radiate at least part of the radiation to the exterior of the radiation body via the radiation exit window, ii) a radiation arrangement configured to provide the radiation, comprising at least one UV radiation source configured to emit UV radiation, iii) a detector arrangement configured to detect an internal radiation intensity of radiation in the radiation body at at least one detector position, and iv) a controller arrangement, functionally coupled to the radiation arrangement and the detector arrangement. For the sake of clarity, it is noted that the indication that the detector arrangement is configured to detect an internal radiation intensity of radiation in the radiation body at at least one detector position may imply in practice that the detector arrangement is capable of obtaining data for calculating and outputting an arbitrary parameter related to the internal radiation intensity at the at least one detector position, wherein it is not necessary that the detector arrangement is capable of determining the actual value of the internal radiation intensity at the at least one detector position.

The controller arrangement of the system according to the invention is configured to set a normal state of the at least one UV radiation source as a default, in which state the at least one UV radiation source provides the UV radiation with an intensity at a maintenance level. Further, the controller arrangement is configured to process input received from the detector arrangement in such a way that occurrence of a disturbance incident followed by a restoration incident can be determined in respect of at least one detector position. This allows the controller arrangement to temporarily set a disinfecting state of the at least one UV radiation source, in which state the at least one UV radiation source provides the UV radiation with an intensity at a level that is increased relative to the maintenance level. In this way, assuming that occurrence of a disturbance incident is indicative of the radiation exit window being contacted by a relatively large object such as a human hand or finger and that occurrence of a subsequent restoration incident is indicative of the object being removed from the radiation exit window, the controller arrangement is configured to implement a measure of temporarily increasing the intensity at which UV radiation is provided exactly when this is appropriate, namely after the radiation exit window has been touched and may be contaminated as a consequence thereof. Apart from that, the intensity at which the UV radiation is provided is kept at a maintenance level, whereby power is saved. The maintenance level of the intensity of the UV radiation may be a zero level, although this is not necessary in the context of the invention. In any case, on the basis of the power-saving measures, the invention provides a way of applying the system in a context in which it is desired to minimize energy consumption without compromising the disinfecting functionality. Practical examples of such a context are a context in which the system is powered by a battery and a context in which the system is powered by a solar cell or another component/system for local energy harvesting. Also, controlling operation of the system in such a way that the intensity of emitted UV radiation is reduced, probably even to zero, during periods that the extent to which the radiation exit window is disinfected may be assumed to be sufficient, contributes to safety of use of the system, and may also involve the advantage of realizing prolonged lifetime of components.

Occurrence of a disturbance incident is determined when it appears that a safety test value that is representative of a parameter of the internal radiation intensity detected by the detector arrangement at the at least one detector position is outside of a safety reference range of values associated with the at least one detector position. According to a first practical option, the safety test value may be representative of a value of the internal radiation intensity of the radiation detected by the detector arrangement. According to a second practical option, the safety test value may be representative of a derivate of the value of the internal radiation intensity of the radiation detected by the detector arrangement, particularly a value of change of the internal radiation intensity in predetermined direction (i.e. to a higher value or a lower value). In respect of the first practical option, the controller arrangement may be configured to assess whether or not a value representing the value of the internal radiation intensity is above or below a predetermined threshold. In respect of the second practical option, the controller arrangement may be configured to calculate a value representing a difference between values of the internal radiation intensity of subsequent detection actions and to assess whether or not the value is larger than a predetermined threshold and/or to calculate a value representing a rate of change of the internal radiation intensity in predetermined direction and to assess whether or not the value is larger than a predetermined threshold. The invention covers various options which may be at the basis of a change of the internal radiation intensity and which are related to the constitution of the system, particularly the features of the radiation body, including the option of such a change resulting from an increase of radiation exiting the radiation body, the option of such a change resulting from an increase of radiation being reflected into the radiation body, and the option of such a change resulting from a combination of a change of exiting radiation and a change of incoming radiation.

The number of detector positions of the detector arrangement can be chosen freely. In this respect, it may be useful to relate the number of detector positions to the size of the radiation exit window and/or to the number of UV radiation sources in case the radiation arrangement comprises more than one UV radiation source. In respect of the latter option, it is explicitly noted that the number of detector positions may or may not be the same as the number of UV radiation sources. In any case, in an embodiment of the system in which the detector arrangement is configured to detect an internal radiation intensity of radiation in the radiation body at at least two different detector positions, there is a safety range of values for each of the detector positions, and the steps of determining at least one safety test value and assessing whether the at least one safety test value is inside or outside of a safety range of values is performed per detector position. In this respect, it is noted that the invention covers both possibilities of the safety range of values being the same and different for two different detector positions. For the sake of completeness, it is noted that in the embodiment of the system as mentioned, i.e. the embodiment in which detection of the internal radiation intensity takes place at more than one detector position, it is possible that the input received from the detector arrangement indicates occurrence of a disturbance incident at more than one detector position. Such input may be representative of the radiation exit window being contacted by an object that is of such a size that the effect thereof on the internal radiation intensity can be found at more than one detector position and/or of the radiation exit window being contacted by a number of objects at the same time.

The invention covers various options relating to duration of a disinfecting period in which the disinfecting state of the at least one UV radiation source is set. According to one option, the disinfecting period is simply of predetermined duration. According to another option, the controller algorithm involves determining duration of the disinfecting period. In the process, the time passed between a disturbance incident and a subsequent restoration incident can be taken into account, wherein it may be so that the duration of the disinfecting period is chosen so as to be longer when the time as mentioned is longer, probably up to a predetermined maximum, or duration of a preceding disinfecting period can be taken into account, wherein it may be so that the duration of the disinfecting period is chosen so as to be longer when the duration of the preceding disinfecting period is shorter than a predetermined minimum duration. Other useful factors which may be taken into account in this respect are also covered by the invention.

The controller algorithm may further involve restoring the normal state of the at least one UV radiation source after the disinfecting period has passed or on occurrence of a disturbance incident during the disinfecting period. The latter is done to guarantee safety by preventing a situation of harmful exposure of a higher organism to the UV radiation. This is especially applicable when the maintenance level of the intensity of the UV radiation is a safe level. If not, it is beneficial if the system is configured to set an adapted state of the at least one UV radiation source at occurrence of a disturbance incident, in which state the at least one UV radiation source provides the UV radiation with an intensity at a level that is decreased relative to the maintenance level, probably at a zero level.

In order to avoid a reaction of the system in situations of the radiation exit window only being shortly/lightly touched, it may be practical if the controller algorithm involves maintaining the normal state of the at least one UV radiation source after occurrence of a disturbance incident followed by a restoration incident if the time passed between the disturbance incident and the restoration incident is less than a predetermined minimum amount.

In an embodiment of the system in which the radiation arrangement comprises at least two UV radiation sources, it is advantageous if the controller algorithm involves temporarily setting a disinfecting state of at least one designated UV radiation source that is configured to emit UV radiation to an area of the radiation body monitored by the detector arrangement at the at least one detector position after occurrence of a disturbance incident followed by a restoration incident. The fact is that setting a disinfecting state of at least one designated UV radiation source is a further power-saving measure when more than one UV radiation source is applied in the system: temporarily increasing the intensity at which UV radiation is provided is not only exactly done when this is appropriate, but also where this is appropriate. Further, the controller algorithm may involve maintaining the normal state of any UV radiation source other than the at least one designated UV radiation source. This is especially applicable when the maintenance level of the intensity of the UV radiation is a zero level. In the case that the maintenance level of the intensity of the UV radiation is a level above zero, it may be so that the controller algorithm involves setting an adapted state of any UV radiation source other than the at least one designated UV radiation source, in which state the UV radiation source provides the UV radiation with an intensity at a level that is decreased relative to the maintenance level, probably to a zero level, as long as the disinfecting state of the designated UV radiation source is set, which may help in keeping the power consumption of the system at the lowest possible level at all times without actually deteriorating the disinfecting functionality of the system.

The invention covers any suitable way of identifying the at least one designated UV radiation source. For example, in case there are at least two detector positions, it may be so that the controller algorithm involves identifying the at least one designated UV radiation source on the basis of a predetermined relation between the UV radiation sources and the detector positions. This implies that it may be so that each of the UV radiation sources is assigned to at least one of the detector positions when it comes to determining which of the UV radiation sources is a designated radiation source on the basis of the input from the detector arrangement. When occurrence of a disturbance incident is found in respect of a detector position, the at least one UV radiation source assigned to the detector position is denoted as being at least one designated UV radiation source and is put to the disinfecting state after occurrence of the subsequent restoration incident.

In an advantageous embodiment of the system according to the invention, the controller arrangement is configured to enable adjustment of the safety reference range of values associated with the at least one detector position, in an automated manner, i.e. by executing a suitable algorithm, and/or on the basis of user input in the case that the system is equipped with a user interface for receiving user input to that end. Further, it is to be noted that the invention also relates to an option of the safety reference range of values associated with the at least one detector position being a fixed system setting. In any case, information representing reference ranges and/or reference rates can be stored in a database or the like, and the controller algorithm can include a step of retrieving such information from the database or the like.

It is suggested earlier that the number of detector positions of the detector arrangement may be chosen freely. The detector arrangement may comprise any suitable number of detectors for realizing the functionality of the detector arrangement as envisaged, wherein the invention includes the possibility of having no more than a single detector in the detector arrangement. The number of UV radiation sources of the radiation arrangement may also be chosen freely. Depending on the constitution of the radiation body and the size of the radiation exit window, it may be advantageous if at least two UV radiation sources are applied in the system and the at least two UV radiation sources are arranged in the radiation body at a distance from each other and/or if the detector arrangement comprises at least two detectors arranged in the radiation body at a distance from each other. Also, it is possible to have more than one detector at one detector position, or nearly the same detector position, wherein it may be practical if the detectors are configured and arranged to detect radiation from different/opposite directions. Likewise, it is possible to have more than one UV radiation source at a certain position, wherein it may be practical if the UV radiation sources are configured and arranged to emit UV radiation in different/opposite directions. It may be practical if the radiation body comprises a slab of material, wherein the at least one UV radiation source and/or the at least one detector is/are embedded in the slab of material. The material of the slab of material may be silicone, for example.

The at least one UV radiation source may be of any suitable type, and may be an LED, particularly a UV-C LED, for example. In such a case, the detector arrangement may comprise the same LED, for example, in view of the fact that it is possible to use a LED as a radiation detector, wherein it is practical if the controller arrangement is configured to put the LED to a detector state instead of a radiation source state from time to time. For example, the LED could be driven to emit radiation during a major percentage such as 90% or 95% of the time and be turned off during the remainder of the time in order to function as detector. In case the radiation arrangement comprises at least two LEDs, it is furthermore possible that the controller arrangement is configured to set the state of LEDs from one group of LEDs to be different from the state of LEDs from another group of LEDs at any given moment. Using a LED both as radiation source and detector offers a possibility to reduce the amount of components needed. In the case of the radiation arrangement comprising at least two LEDs, it may further be so that accuracy of determining locations of touch on the radiation exit window and identifying the designated UV radiation source(s) is improved.

In the context of the invention, it is possible that the detector arrangement is configured to detect an internal radiation intensity of the UV radiation. Another option is that the system comprises another type of radiation source besides the UV radiation sources, namely at least one radiation source configured to provide radiation of distinctly longer wavelengths, such as visible light or infrared radiation, and that the detector arrangement is configured to at least detect the internal radiation intensity of the radiation of the distinctly longer wavelengths. In view of the fact that absorption of radiation of longer wavelengths is typically significantly lower, the step of checking whether or not a disturbance incident and a subsequent restoration incident occur at the at least one detector position can be performed with high accuracy, while the number of additional radiation sources and detectors can be kept limited.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

The above-described and other aspects of the invention will be apparent from and elucidated with reference to the following detailed description of practical embodiments of a system comprising i) a radiation body comprising a radiation exit window, wherein the radiation body is configured to receive radiation that at least comprises UV radiation, and to radiate at least part of the radiation to the exterior of the radiation body via the radiation exit window, ii) a radiation arrangement configured to provide the radiation, comprising at least one UV radiation source configured to emit UV radiation, iii) a detector arrangement configured to detect an internal radiation intensity of radiation in the radiation body at at least one detector position, and iv) a controller arrangement, functionally coupled to the radiation arrangement and the detector arrangement.

### BRIEF DESCRIPTION OF THE DRAWINGS

The invention will now be explained in greater detail with reference to the figures, in which equal or similar parts are indicated by the same reference signs, and in which:
Fig. 1 diagrammatically shows a system according to a first embodiment of the invention,
Fig. 2 diagrammatically shows a portion of a system according to a second embodiment of the invention, and
Fig. 3 diagrammatically shows a number of objects which can be equipped with a system according to the invention.

### DETAILED DESCRIPTION OF THE EMBODIMENTS

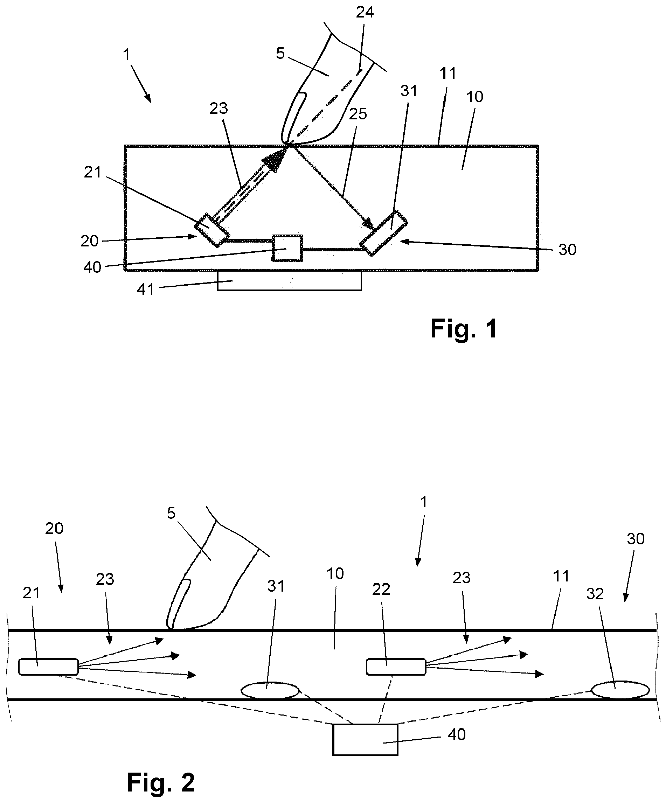

Fig. 1 diagrammatically shows a system 1 according to a first embodiment of the invention.

The system 1 comprises a radiation body 10, a radiation arrangement 20, a detector arrangement 30 and a controller arrangement 40.

The radiation body 10 comprises a radiation exit window 11. Fig. 1 illustrates the practical options of the radiation body 10 comprising a slab of material and the radiation exit window 11 being of generally flat appearance.

The radiation arrangement 20 is configured to emit radiation that at least comprises UV radiation. In the shown example, the radiation arrangement 20 comprises a UV radiation source 21 configured to emit UV radiation, in one or more directions, at least towards an interior side of the radiation exit window 11. In Fig. 1, the UV radiation is depicted by means of an arrow 23. A practical example of the UV radiation source 21 is a UV-C LED. Fig. 1 illustrates the practical option of the UV radiation source 21 being embedded in the slab of material of the radiation body 10, wherein the slab of material is transparent to the UV radiation 23 provided by the UV radiation source 21.

The detector arrangement 30 is configured to detect an internal radiation intensity in the radiation body 10. Fig. 1 illustrates the practical option of the detector arrangement 30 comprising a detector 31 that is embedded in the slab of material of the radiation body 10.

The controller arrangement 40 is functionally coupled to the radiation arrangement 20 and the detector arrangement 30 and is configured to control operation of the system 1. The invention covers both an option of the system 1 comprising a communication arrangement configured to enable data communication between the controller arrangement 40 and the radiation arrangement 20 and the detector arrangement 30, respectively, to take place in a wired fashion and an option of the system 1 comprising a communication arrangement configured to enable the data communication to take place in a wireless fashion, and also covers an option of the system 1 comprising a communication arrangement configured to enable the data communication to take place partly in a wired fashion and partly in a wireless fashion. Fig. 1 illustrates the practical option of the controller arrangement 40 comprising a unit that is embedded in the slab of material of the radiation body 10. That does not alter the fact that it is also possible that additionally or alternatively, the controller arrangement 40 comprises at least one unit that is positioned outside of the slab of material of the radiation body 10.

The radiation arrangement 20, the detector arrangement 30 and the controller arrangement 40 may be powered in any suitable way. The invention is especially applicable to situations in which only a limited amount of power is available, which is the case in situations in which it is not practical to have a connection to the mains. In view thereof, electric coupling of the radiation arrangement 20, the detector arrangement 30 and the controller arrangement 40 to a battery 41 is mentioned as an example, which does not alter the fact that the invention is not restricted to any particular power supply.

The radiation exit window 11 of the radiation body 10 is configured to transmit part of the UV radiation 23 of the UV radiation source 21. In Fig. 1, the radiation exiting the radiation body 10 through the radiation exit window 11 is indicated by means of a dashed line 24. The detector 31 is at a position of receiving internal radiation 25 emanating from the radiation exit window 11 inward into the slab of material of the radiation body 10, and is configured to provide an output signal to the controller arrangement 40 representing intensity of the internal radiation 25. Depending on the structure of the radiation exit window 11, the internal radiation 25 may be obtained through at least one of i) scattering of the UV radiation 23 radiated by the UV radiation source 21 at the position of the radiation exit window 11 and ii) reflection of the UV radiation 23 radiated by the UV radiation source 21 on the interior side of the radiation exit window 11. It may be so that scattering of the UV radiation 23 radiated by the UV radiation source 21 especially takes place when an object 5 such as a person's finger is present on an exterior side of the radiation exit window 11, as diagrammatically shown in Fig. 1. In any case, when the radiation exit window 11 is contacted by an object 5, both the exiting radiation 24 and the internal radiation 25 traveling from the interior side of the radiation exit window 11 towards the detector 31 will likely change.

The main purpose of having the radiation arrangement 20 in the system 1 is keeping the exterior side of the radiation exit window 11 in a disinfected state. When bacteria or viruses end up on the exterior side of the radiation exit window 11, the bacteria or viruses are killed or at least rendered inactive under the influence of the exiting radiation 24 at the position of the bacteria or viruses. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or animals/pets.

The controller arrangement 40 is configured to control the radiation arrangement 20 in dependence of the signal provided by the detector arrangement 30, especially when it comes to setting the intensity of the UV radiation 23 emitted by the UV radiation source 21. The fact is that the controller arrangement 40 is configured to set a normal state of the UV radiation source 21 in which the UV radiation source 21 provides the radiation 23 with an intensity at a maintenance level as a default, and to temporarily set a disinfecting state of the UV radiation source 21 in which the UV radiation source 21 provides the UV radiation 23 with an intensity at a level that is increased relative to the maintenance level after occurrence of a disturbance incident involving a change of the output signal of the detector 31 and a subsequent restoration incident. As explained earlier, the output signal of the detector 31 represents the intensity of the internal radiation 25. In this respect, it is noted that occurrence of a disturbance incident is determined when at least one test value that is representative of a parameter of the intensity of the internal radiation 25 shifts from inside a safety reference range of values to outside of the safety range of values, and that occurrence of a restoration incident is determined when a shift of the at least one test value in opposite direction is found. In order to ensure proper functioning of the system 1, the references relied upon in assessing occurrence of a disturbance incident are preferably chosen such that occurrence of a disturbance incident is not determined when relatively small objects are present on the radiation exit window 11, particularly objects having dimensions of tens to hundreds micrometer or even smaller dimensions, such as tiny droplets and/or grease/dirt containing bacteria and/or viruses, but only when relatively large objects are present on the radiation exit window 11.

The invention provides a way of operating the system 1 which is suitable for realizing a disinfected state of the radiation exit window 11 on the one hand, so that there is no health risk to a human beings or higher animals touching the radiation exit window 11, and only requiring a minimum amount of electrical power on the other hand. In view of the fact that contamination of the radiation exit window 11 is caused by the very event of touch on the radiation exit window 11, the invention proposes to provide a relatively high dose of UV radiation 23 after such an event has taken place so as to restore the disinfected state of the radiation exit window 11 as fast as possible, and to have a maintenance level of the intensity of the UV radiation 23 apart from that. In this way, any microorganisms as may have been transferred to the radiation exit window 11 during an event of touch are killed or at least rendered inactive shortly after the event has taken place, after which the system 1 switches back to a maintenance mode, until a new event of touch takes place. The time duration of the disinfecting state of the UV radiation source 21 may be of predetermined length or may be determined on the basis of one or more relevant factors such as time passed between a disturbance incident and a subsequent restoration incident and/or duration of a preceding disinfecting period.

Any appropriate number of UV radiation sources 21 can be chosen in the context of the invention, wherein it is possible to have an arrangement of the UV radiation sources 21 in which the UV radiation sources 21 are positioned to radiate the UV radiation 23 to respective areas of the radiation exit window 11 of the radiation body 10, whether or not with overlap. Also, any appropriate number of detectors 31 can be chosen in the context of the invention. In this respect, it is noted that Fig. 2 diagrammatically shows a portion of a system 2 according to a second embodiment of the invention, which system 2 includes a plurality of UV radiation sources 21 and a plurality of detectors 31. In view thereof, the system 2 will be referred to as extended system 2. The extended system 2 may in fact be regarded as comprising a number of the above-described systems 1 according to the first embodiment of the invention, in an integrated fashion, wherein the number may be chosen to have any appropriate value. For the sake of illustration, a portion of the extended system 2 including two radiation source 21, 22 and two detectors 31, 32 is shown in Fig. 2. The UV radiation sources 21, 22 can be arranged in any appropriate pattern, including a pattern in which the UV radiation sources 21, 22 are equally distributed throughout the slab of material of the radiation body 10, in an imaginary plane extending parallel to the radiation exit window 11, in a row or in a 2D array. In any case, it may be practical if the UV radiation sources 21, 22 are arranged at a distance from each other. The same is applicable to the detectors 31, 32.

A general explanation of how the extended system 2 works is now provided. First, it is to be noted that most materials have a high absorption for radiation in the UV-C wavelength ranges. For example, silicone materials typically absorb over 20% of the radiation, per cm travelled. As a result, after 10 cm, only (1-0.2)¹⁰ = about 10% of the radiation is left, and after 20 cm, this has reduced further to only about 1%. If it is assumed that the material of the slab of material of the radiation body 10 is silicone, that the UV radiation sources 21, 22 are arranged in a row, at a distance of 10 cm from each other, and that the UV radiation detectors 31, 32 are also arranged in a row, at a distance of 10 cm from each other, at intermediate positions between the UV radiation sources 21, 22 as seen in the direction in which the row extends, it is found that roughly speaking, the intensity of the internal radiation 25 at each of the detector positions is mainly determined by the UV radiation 23 of the nearest UV radiation source 21, 22 in view of the fact that a percentage of the UV radiation 23 of the nearest UV radiation source 21, 22 that is left at a detector position is about 33%, while a percentage of the UV radiation 23 of the second-nearest UV radiation source 21, 22 that is left at the same detector position is only about 3.5%.

As explained in the foregoing, when the radiation exit window 11 of the radiation body 10 is touched by an object 5 such as a person's finger, as diagrammatically shown in Fig. 2, this involves a local change of the intensity of the internal radiation 25. As a further power-saving measure, when the radiation arrangement 20 comprises more than one UV radiation source 21, 22, as is the case in the extended system 2, the controller arrangement 40 is configured to temporarily set a disinfecting state of only the at least one UV radiation source 21, 22 that is configured and arranged to emit UV radiation 23 to an area that is determined to be an area that has been subjected to touch through determination of occurrence of a disturbance incident and a subsequent restoration incident at one or more detector positions. On the basis of the absorption of UV radiation 23 in the material of the radiation body 10, as explained in the foregoing, and on the basis of the way in which the detector arrangement 30 is designed, probably with a distributed pattern of detector positions throughout the slab of material of the radiation body 10, it is very well possible to determine which of the UV radiation sources 21, 22 can be identified as a designated UV radiation source 21, 22 that is to be temporarily put to the disinfecting state in order to eliminate the possible health risk following from a recent event of touch on the radiation exit window 11, in the very area that has been involved in the event. For example, it may be so that a predetermined relation between the UV radiation sources 21, 22 on the one hand and the at least one detector position on the other hand is stored in the controller arrangement 40, and that the controller arrangement 40 is configured to identify the one or more designated UV radiation sources 21, 22 by applying the input from the detector arrangement 30 to the predetermined relation. This means that every time a disturbance incident occurs at a detector position, one or more UV radiation sources 21, 22 are automatically identified as being designated UV radiation sources 21, 22.

In respect of temporarily setting the disinfecting state of one or more designated UV radiation sources 21, 22, it is noted that in case the maintenance level of the intensity of the UV radiation 23 is not a zero level, it is possible to set an adapted state of any UV radiation source 21, 22 other than the designated UV radiation source(s) 21, 22, in which state the UV radiation source(s) 21, 22 concerned provide(s) the UV radiation 23 with an intensity at a level that is decreased relative to the maintenance level, as long as the disinfecting state of the designated UV radiation source(s) 21, 22 is set, which may be beneficial from an energy-saving point of view and/or in view of the total available power at any given time.

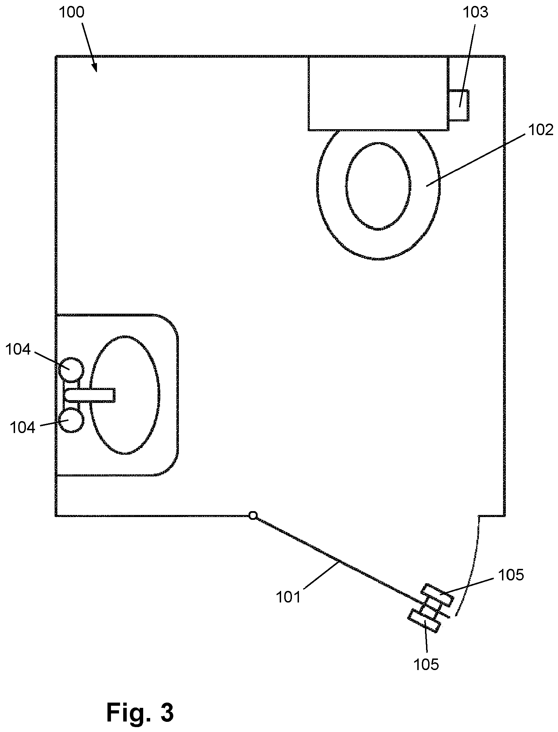

Fig. 3 diagrammatically shows a number of objects which can be equipped with the system 1, 2 according to the invention, particularly objects as can be found in a bathroom 100 and on a bathroom door 101, namely a toilet seat 102, a toilet flushing knob 103, tap knobs 104 and the door knobs 105 of the bathroom door. The shown objects are only a few of the many objects included in the scope of protection of the invention. The object 102, 103, 104, 105 can be of conventional design, in which case the system 1, 2 or at least the radiation body 10 of the system 1, 2 and components arranged in the radiation body 10 can be arranged on an exterior surface of the object 102, 103, 104, 105, or the object 102, 103, 104, 105 can be of adapted design, in which case the system 1, 2 or at least the radiation body 10 of the system 1, 2 and components arranged in the radiation body 10 can have a sunk arrangement in a member of the object 102, 103, 104, 105, for example, wherein the radiation exit window 11 of the radiation body 10 of the system 1, 2 can be flush with a surrounding part of an original exterior surface of the object 102, 103, 104, 105.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

The invention covers the option that the normal state of the at least one UV radiation source 21, 22 that is set as a default is one of at least two states involving a relatively low level of the intensity of the UV radiation 23 as the maintenance level of the intensity of the UV radiation 23. For example, when the invention is applied in an environment in which events of touch on the radiation exit window 11 are normally only expected during the day and not during the night, it may be so that during the night, a normal state of the at least one UV radiation source 21, 22 is set in which the intensity of the UV radiation 23 is at a zero level, and that during the day, a normal state of the at least one UV radiation source 21, 22 is set in which the level of the intensity of the UV radiation 23 is somewhat higher.

Notable aspects of the invention are summarized as follows. In a system 1, 2 comprising a radiation body 10, a radiation arrangement 20 configured to provide radiation at least comprising UV radiation 23 to the radiation body 10, a detector arrangement 30 configured to detect an internal radiation intensity of radiation 25 in the radiation body 10 at at least one detector position, and a controller arrangement 40 that is functionally coupled to the radiation arrangement 20 and the detector arrangement 30, the controller arrangement 40 is configured to apply a controller algorithm designed to control the radiation arrangement 20 in dependence on input from the detector arrangement 30. In particular, when the input from the detector arrangement 30 indicates a change of the internal radiation intensity that is qualified as a disturbance incident at the at least one detector position, and that can be assumed to follow from an event of touch on a radiation exit window 11 of the radiation body 10, an action of temporarily changing a state of at least one UV radiation source 21, 22 of the radiation arrangement 20 from a maintenance state to a disinfecting state of increased intensity of the UV radiation 23 is performed. By setting the disinfecting state of at least one UV radiation source 21, 22 only after an event of touch has taken place, and, in case the radiation arrangement 20 comprises a plurality of UV radiation sources 21, 22, only doing so in respect of the one or more radiation sources 21, 22 which are located to irradiate the area involved in the event of touch, disinfection of the radiation exit window 11 is realized at only a minimum amount of electric energy.

## Claims

1. A system (1, 2) comprising:
- a radiation body (10) comprising a radiation exit window (11), wherein the radiation body (10) is configured to receive radiation that at least comprises UV radiation (23), and to radiate at least part of the radiation to the exterior of the radiation body (10) via the radiation exit window (11);
- a radiation arrangement (20) configured to provide the radiation, comprising at least one UV radiation source (21, 22) configured to emit UV radiation (23);
- a detector arrangement (30) configured to detect an internal radiation intensity of radiation (25) in the radiation body (10) at at least one detector position; and
- a controller arrangement (40), functionally coupled to the radiation arrangement (20) and the detector arrangement (30), configured to set a normal state of the at least one UV radiation source (21, 22) as a default, in which state the at least one UV radiation source (21, 22) provides the UV radiation (23) with an intensity at a maintenance level, and configured to receive input from the detector arrangement (30) and to apply a controller algorithm that involves i) determining at least one safety test value that is representative of a parameter of the internal radiation intensity detected by the detector arrangement (30) at the at least one detector position, ii) assessing whether the at least one safety test value is inside or outside of a safety range of values associated with the at least one detector position, and iii) after occurrence of a disturbance incident involving a shift of the at least one safety test value from inside the safety reference range of values to outside of the safety reference range of values and a subsequent restoration incident involving a shift of the at least one test value in opposite direction, temporarily setting a disinfecting state of the at least one UV radiation source (21, 22), in which state the at least one UV radiation source (21, 22) provides the UV radiation (23) with an intensity at a level that is increased relative to the maintenance level.

2. The system (1, 2) according to claim 1, wherein the controller algorithm involves determining duration of a disinfecting period in which the disinfecting state of the at least one UV radiation source (21, 22) is set in relation to time passed between a disturbance incident and a subsequent restoration incident and/or in relation to duration of a preceding disinfecting period.

3. The system (1, 2) according to claim 2, wherein the controller algorithm involves restoring the normal state of the at least one UV radiation source (21, 22) after the disinfecting period has passed or on occurrence of a disturbance incident during the disinfecting period.

4. The system (1, 2) according to any of claims 1-3, wherein the controller algorithm involves maintaining the normal state of the at least one UV radiation source (21, 22) after occurrence of a disturbance incident followed by a restoration incident if the time passed between the disturbance incident and the restoration incident is less than a predetermined minimum amount.

5. The system (1, 2) according to any of claims 1-4, wherein the maintenance level of the intensity of the UV radiation (23) is a zero level.

6. The system (1, 2) according to any of claims 1-5, wherein the at least one safety test value is representative of a value of the internal radiation intensity of the radiation (25) detected by the detector arrangement (30) and/or a value of change in predetermined direction of the internal radiation intensity of the radiation (25) detected by the detector arrangement (30).

7. The system (2) according to any of claims 1-6, wherein the radiation arrangement (20) comprises at least two UV radiation sources (21, 22), and wherein the controller algorithm involves temporarily setting a disinfecting state of at least one designated UV radiation source (21, 22) that is configured to emit UV radiation (23) to an area of the radiation body (10) monitored by the detector arrangement (30) at the at least one detector position after occurrence of a disturbance incident followed by a restoration incident.

8. The system (2) according to claim 7, wherein the controller algorithm involves maintaining the normal state of any UV radiation source (21, 22) other than the at least one designated UV radiation source (21, 22).

9. The system (2) according to claim 7, wherein the maintenance level of the intensity of the UV radiation (23) is a level above zero, and wherein the controller algorithm involves setting an adapted state of any UV radiation source (21, 22) other than the at least one designated UV radiation source (21, 22), in which state the UV radiation source (21, 22) provides the UV radiation (23) with an intensity at a level that is decreased relative to the maintenance level, as long as the disinfecting state of the designated UV radiation source (21, 22) is set.

10. The system (2) according to any of claims 7-9, wherein the detector arrangement (30) is configured to detect an internal radiation intensity of radiation (25) in the radiation body (10) at at least two detector positions, and wherein the controller algorithm involves identifying the at least one designated UV radiation source (21, 22) on the basis of a predetermined relation between the UV radiation sources (21, 22) and the detector positions.

11. The system (1, 2) according to any of claims 1-10, wherein the radiation body (10) comprises a slab of material, and wherein the at least one UV radiation source (21, 22) of the radiation arrangement (20) is embedded in the slab of material and/or at least one detector (31, 32) of the detecting arrangement (30) is embedded in the slab of material.

12. The system (1, 2) according to any of claims 1-11, comprising at least one radiation source configured to provide radiation of distinctly longer wavelengths than UV wavelengths, wherein the detector arrangement (30) is configured to at least detect the internal radiation intensity of the radiation of the distinctly longer wavelengths.

13. An object (102, 103, 104, 105) comprising the system (1, 2) according to any of claims 1-12, wherein the radiation exit window (11) of the radiation body (10) of the system (1, 2) is arranged to be at an exterior side of the object (102, 103, 104, 105).

14. The object (102, 103, 104, 105) according to claim 13, selected from a group comprising domestic appliances, furniture, construction elements of buildings and vehicles, sanitary parts, medical devices, and components of all of the foregoing.

15. Method of adding a system (1, 2) according to any of claims 1-12 to an existing object (102, 103, 104, 105), wherein the radiation body (10) of the system (1, 2) is applied to an exterior surface of the existing object (102, 103, 104, 105).
